# EUROPÄISCHE PATENTSCHRIFT

(11) **EP 0 732 321 B1**
(45) Veröffentlichungstag und Bekanntmachung des Hinweises auf die Patenterteilung: **14.10.1998**
(21) Anmeldenummer: 96100624.4
(22) Anmeldetag: 18.01.1996
(51) Int. Cl.: C07C 67/307, C07C 69/65

(54) **Verfahren zur Herstellung von 2-Aryl-2-chlormalonsäurediestern**
Process for the preparation of diesters of 2-aryl-2-chloromalonic acid
Procédé de fabrication de diesters d'acide 2-aryle-2-chloromalonique

(30) Priorität: 15.03.1995 DE 19509377
(43) Veröffentlichungstag der Anmeldung: 18.09.1996
(73) Patentinhaber: HÜLS AKTIENGESELLSCHAFT, 45764 Marl (DE)
(72) Erfinder: Theis, Christoph, Dr., D-53859 Niederkassel (DE); Bordeianu, Radu, Dr., D-45772 Marl (DE); Latz, Wilfried, D-53859 Niederkassel (DE)

(56) Entgegenhaltungen:
- DE-A- 1 948 475
- DE-A- 4 420 263
- JOURNAL OF THE CHEMICAL SOCIETY, 1928, LETCHWORTH GB, Seiten 1607-1616, XP000573075 FLUERSCHEIM B. ET AL.: "The Laws of Aromatic Substitution.Part VIII."

## Beschreibung

Die vorliegende Erfindung betrifft ein neues Verfahren zur Herstellung von 2-Aryl-2-chlormalonsäurediestern aus 2-Arylmalonsäurediestern.

2-Aryl-2-chlormalonsäurediester werden nach der deutschen Patentanmeldung mit dem Aktenzeichen DE P 44 15 872.6 als hochwirksame Aktivatoren bei der Herstellung von Ethylencopolymerisaten eingesetzt.

Für die Herstellung von 2-Aryl-2-chlormalonsäurediestern sind bereits verschiedene Methoden angegeben worden. So erhält Flürscheim, J. Chem. Soc. 1928, 1607-16, 2-Chlor-2-phenylmalonsäurediethylester durch Umsetzung von 2-Phenylmalonsäurediethylester mit trockenem Chlorgas in Tetrachlorkohlenstoff bei 0 °C in mäßiger Ausbeute. Robert, Tetrahedron 42, 2275-81 (1986), stellt 2-Chlor-2-phenylmalonsäurediester durch Umsetzung von geminalen Dicyanoverbindungen mit Chlorwasserstoff dar. Citterio, J. Chem. Research (S), 156-157 (1988), oxidiert 2-Phenylmalonsäurediester mit Mangan (III)-acetat in Gegenwart von Chloridionen und erhält dabei 2-Chlor-2-phenylmalonsäurediester.

In DE-A-19 48 475 werden u. a. 2,2-Dichlormalonsäureester hergestellt. Dabei ist der als Lösemittel eingesetzte Alkohol unter den gegebenen Bedingungen nicht inert. Die auch angegebenen Verfahren zur Herstellung der entsprechenden Monochlorverbindungen sind wegen geringer Ausbeuten und Reinheiten ohne technische Bedeutung.

Allen in der Literatur beschriebenen Verfahren ist gemein, daß die Ausbeuten und Reinheiten der gewünschten Produkte meist nur unbefriedigend sind und die Ausgangsmaterialien teilweise nur schwierig und aufwendig erhalten werden. Zudem werden teilweise Chlorierungsmittel eingesetzt, die aufgrund ihrer Natur oder ihrer bei der gewünschten Umsetzung entstehenden Reaktionsprodukte aus verfahrenstechnischer, wirtschaftlicher und ökologischer Sicht für eine industrielle Fertigung der gewünschten Verbindungen wenig geeignet sind.

Es bestand daher die Aufgabe, ein verfahrenstechnisch einfaches, wirtschaftliches und vor allen Dingen ergiebiges Verfahren zur Herstellung von 2-Aryl-2-chlormalonsäurediestern aus den zugrundeliegenden und bequem zugänglichen 2-Arylmalonsäurediestern bereitzustellen.

Die Aufgabe wird überraschend dadurch gelöst, daß man 2-Arylmalonsäurediester mit wäßrigem Hypochlorit in Gegenwart eines inerten Lösevermittlers ausgewählt aus Acetonitril, Propionitril oder Butyronitril bei pH 8 bis 14 umsetzt.

Die gewünschten 2-Aryl-2-chlormalonsäurediester haben die allgemeine Formel I

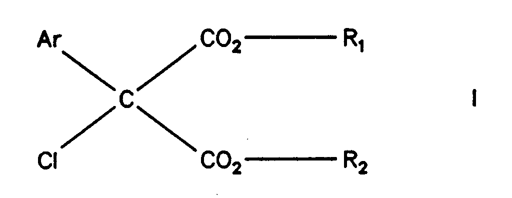

Darin stehen R₁ und R₂ unabhängig voneinander für gradkettiges oder verzweigtes Alkyl mit 1 bis 6 C-Atomen, Cycloalkyl oder Aralkyl. Ar bedeutet Phenyl oder Aryl, bei dem am Aromaten Alkylreste mit 1 bis 4 C-Atomen oder Halogenatome stehen.

In den 2-Arylmalonsäurediestern der allgemeinen Formel II 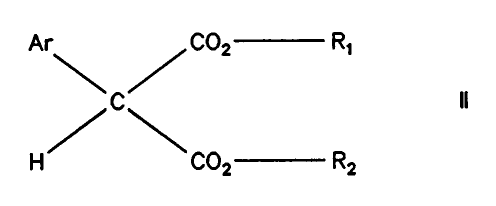 haben die Substituenten R₁, R₂ und Ar die vorgenannten Bedeutungen.

Unter wäßrigem Hypochlorit werden wäßrige Lösungen und wäßrige Suspensionen von Salzen der hypochlorigen Säuren verstanden. Erdalkalihypochlorite werden meist als Suspension und Alkalihypochlorite als Lösung eingesetzt. Dabei handelt es sich beispielsweise um die Calcium-, Barium-, Natrium- oder Kaliumsalze. Vorzugsweise werden Alkalihypochlorit-Lösungen für die Reaktion verwendet.

Als inerte Lösevermittler ausgewählt aus Acetonitril, Propionitril oder Butyronitril verwendet man vorzugsweise Acetonitril.

Zusätzlich können auch andere, z.B. mit Wasser nicht mischbare Hilfssolventien eingesetzt werden. Die Menge an einzusetzendem Lösevermittler ist an sich nicht kritisch, da nach Reaktionsende im allgemeinen ein gut trennbares Zweiphasengemisch resultiert und sich der Lösevermittler sehr leicht sowohl aus der erhaltenen organischen wie auch aus der wäßrigen Phase, beispielsweise durch Destillation, zurückgewinnen und ohne weitere Reinigung zurückführen läßt.

Die aufgefundene Umsetzung kann beispielsweise bei Verwendung einer Natriumhypochlorit-Lösung durch folgende Reaktionsgleichung beschrieben werden:

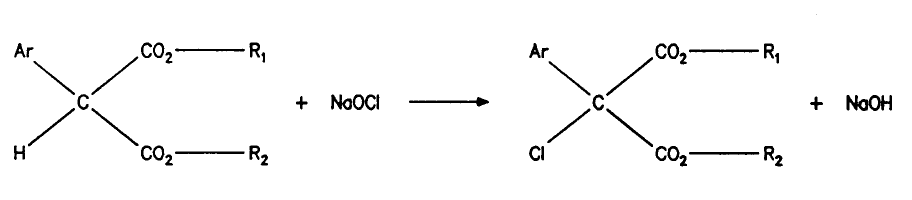

Überraschend wurde gefunden, daß es für eine hohe Ausbeute an 2-Aryl-2-chlormalonsäurediester und für eine hohe Produktreinheit wesentlich ist, daß die bei der Reaktion freiwerdenden Lauge durch eine Säure abgepuffert wird. Vorzugsweise stellt man dabei einen pH-Wert von 11 bis 14 ein.

Geeignete Säuren zur Abpuffung sind ggf. verdünnte Mineralsäure, wie Salz-, Schwefel- oder Phosphorsäure, gasförmige Säuren, wie beispielsweise Chlorwasserstoff und Kohlensäure, organische Säuren, wie Ameisensäure oder Mono-, Di- und Trichloressigsäure und -propionsäure und auch aromatische Carbonsäuren, die mit Wasser verdünnt sein können und die einen oder mehrere inerte Substituenten tragen können.

Desweiteren wurde gefunden, daß niedrige Reaktionstemperaturen die erzielbaren Ausbeuten und Produktreinheiten begünstigen. Es wird deshalb zweckmäßigerweise im Temperaturbereich 0 bis 50 °C, vorzugsweise im Temperaturbereich 2 bis 25 °C, gearbeitet. Dieser Temperaturbereich gewährleistet eine rasche und schonende Umsetzung der eingesetzten Edukte. Die Anwendung noch tieferer Temperaturen ist aber auch möglich.

Bei der Chlorierung von 2-Arylmalonsäurediestern mit Hypochlorit kann ein molares Verhältnis von 1 : 1 eingestellt werden. Zweckmäßigerweise wird Hypochlorit jedoch im Überschuß angewandt. Das 2-Arylmalonsäurediester/Hypochlorit-Molverhältnis liegt daher vorzugsweise im Bereich von 1 : 1 bis 1 : 5. Besonders bevorzugt liegt das Verhältnis bei 1 : 1 bis 1 : 2 und ganz besonders bevorzugt bei 1 : 1,05 bis 1 : 1,2. Nach Reaktionsende kann der ggf. noch vorhandene Hypochloritanteil in an sich bekannter Weise, etwa durch Zugabe von wäßrigem Natriumsulfit, zerstört werden.

Das erfindungsgemäße Verfahren liefert die 2-Aryl-2-chlormalonsäurediester in hohen Ausbeuten und hoher Reinheit. Die dazu benötigten Chemikalien sind preiswert, gut handhabbar und physiologisch unbedenklich.

Das erfindungsgemäße Verfahren kann beispielsweise so durchgeführt werden, daß das Hypochlorit in Form einer wäßrigen Lösung oder Suspension vorgelegt und das Edukt, ggf. verdünnt mit da inerten Lösevermittler, unter Konstanthaltung des pH-Bereiches durch parallele Dosierung einer Säure zudosiert wird, wobei die Säuredosierung auch in der Nachreaktionsphase zur Aufrechterhaltung des gewählten pH-Bereiches fortgesetzt wird. Es kann aber auch so verfahren werden, daß zum vorgelegten Hypochlorit zunächst der inerte Lösevermittler zugegeben und dann das unverdünnte Edukt unter pH-Konstanthaltung zudosiert wird. Ferner ist es möglich, Edukt und gewählte Säure gemeinsam, ggf. in Verbindung mit dem Lösevermittler, zu dosieren. Es kann aber auch eine inverse Arbeitsweise, bei der Edukt und Lösevermittler vorgelegt und Hypochloritlösung und parallel Säure dosiert wird, gewählt werden.

Im allgemeinen resultiert bei diesen Umsetzungen ein zweiphasiges Reaktionsgemisch, aus dem die organische Phase leicht abgetrennt werden kann. Die erhaltene organische Phase enthält im allgemeinen den größten Teil der eingesetzten inerten Lösevermittler. Diese können - wie auch aus der verbliebenen wäßrigen Phase - hieraus durch eine einfache Destillation abgetrennt werden.

Das gewünschte Produkte wird gleichfalls in an sich bekannter Weise, z. B. durch fraktionierende Vakuumdestillation, in hochreiner Form erhalten.

Die nachfolgenden Beispiele sollen die Erfindung verdeutlichen.

### Beispiel 1

217,8 g wäßrige Natriumhypochlorit-Lösung (Gehalt: 8,55 % NaOCl entsprechend 500 mMol NaOCl) werden auf 12 °C abgekühlt. Der pH-Wert der Lösung wird durch Zugabe wäßriger 20%iger Salzsäure auf ca. 13 eingestellt. Zu dieser gekühlten Vorlage werden 65,0 g Acetonitril zugesetzt und dann im Verlauf von ca. 30 Minuten 106,2 g 2-Phenylmalonsäurediethylester [450 mMol] zugetropft, wobei der pH-Wert der Reaktionsmischung durch gleichzeitiges Dosieren der wäßrigen 20%igen Salzsäure im Bereich 13,9 bis 13,2 und die Innentemperatur durch Kühlung bei ca. 12 °C gehalten wird.

Nach beendeter Malonester-Dosierung läßt man unter Konstanthaltung des pH-Bereiches ca. 120 Minuten nachreagieren. Dann wird vorhandenes, überschüssiges Hypochlorit durch Zugabe von Natriumsulfit zerstört. Aus dem resultierenden 2-Phasen-Gemisch wird die organische Phase abgetrennt.

Nach Eindampfen der organischen Phase wird der dann resultierende Rückstand einer Flash-Vakuum-Destillation unterworden: Man erhält 116,4 g 2-Chlor-2-phenylmalonsäurediethylester mit dem Siedepunkt 106-8 °C [0,2 hPa]. Das entspricht einer Ausbeute von 95,6 % d. Th. Die gaschromatographische ermittelte Reinheit des Produktes beträgt ≥ 98,5 % (Edukt-Gehalt: ≤ 0,3 %).

### Beispiel 2

Es wird wie in Beispiel 1 verfahren, dabei wird jedoch ein Anfangs- und Reaktions-pH-Bereich von 11,0 bis 11,2 eingestellt.

Nach analoger Aufarbeitung werden 110,9 g Destillat erhalten. Das entspricht einer Ausbeute von 91,1 % d. Th. Der durch GC ermittelte Produkt-Gehalt beträgt 95,3 % (Edukt-Gehalt: 3,7 %).

### Beispiel 3

Es wird wie in Beispiel 1 verfahren. Dabei werden jedoch 131,4 [450 mMol] 2-Phenylmalonsäuredi-n-butylester und 100,0 g Acetonitril eingesetzt. Die Edukt-Dosierzeit beträgt ca. 60 Minuten.

Das durch analoge Aufarbeitung erhaltene acetonitrilfreie Rohprodukt wird an einer 20 cm-Vigreux-Kolonne einer fraktionierenden Vakuum-Destillation unterworfen. Man erhält 128,9 g 2-Chlor-2-phenylmalonsäuredin-n-butylester mit dem Siedepunkt 128-30 °C [0,2 hPa]. Das entspricht einer Ausbeute von 80,8 % d. Th., Reinheit (GC): > 98 %.

### Beispiel 4

Es wird analoge zu Beispiel 3 verfahren. Dabei werden jedoch 118,0 g [450 mMol] 2-Phenylmalonsäuredi-n-propylester eingesetzt. Die Edukt-Dosierzeit beträgt ca. 120 Minuten. Der Anfangs- und Reaktions-pH-Bereich liegt bei 13,4 - 13,6. Das in analoger Weise erhaltene Produkt wird wie in Beispiel 3 aufgearbeitet. Es werden 124,7 g [84,9 % Ausbeute] 2-Chlor-2-phenylmalonsäuredi-n-propylester mit einem per GC ermittelten Gehalt von > 98 % erhalten.

### Beispiel 5

Man verfährt analog zu Beispiel 4. Es werden jedoch 93,6 g [450 mMol] 2-Phenylmalonsäuredimethylester im Gemisch mit 100,0 g Acetonitril im Verlauf von ca. 2 Stunden dosiert. Durch Aufarbeitung analog Beispiel 3 werden 94,2 g Reinprodukt erhalten, die Ausbeute an 2-Chlor-2-phenylmalonsäuredimethylester entspricht 86,3 % der Theorie, Gehalt: > 98 %.

### Beispiel 6

Man verfährt wie in Beispiel 1, setzt aber 120,0 g Acetonitril und 112,5 g [475 mMol] 2-(p-Toluyl)-malonsäurediethylester ein. Die Nachreaktionszeit beträgt 2,5 Stunden bei ca. 15 °C. Das nach analoger Aufarbeitung erhaltene acetonitrilfreie Rohprodukt wird gemäß Beispiel 3 destillativ gereinigt. Man erhält 119,8 g 2-Chlor-2-(p-toluyl)-malonsäurediethylester (Gehalt: > 98,5 %). Das entspricht einer Ausbeute von 92,9 % der Theorie.

### Beispiel 7

Es wird analog zu Beispiel 6 verfahren. Man setzt jedoch 121,7 g [450 mMol] 2-(p-Chlorphenyl)-malonsäurediethylester ein. Dabei werden 130,9 g 2-Chlor-2-(p-chlorphenyl)-malonsäurediethylester (Gehalt: > 98 %) erhalten. Dies entspricht einer theoretischen Ausbeute von 95,4 %.

## Patentansprüche

1. Verfahren zur Herstellung von 2-Aryl-2-chlormalonsäurediestern aus 2-Arylmalonsäurediestern,
dadurch gekennzeichnet,
daß man die 2-Arylmalonsäurediester mit wäßrigem Hypochlorit in Gegenwart eines inerten Lösevermittlers ausgewählt aus Acetonitril, Propionitril oder Butyronitril bei pH 8 bis 14 umsetzt.

2. Verfahren nach Anspruch 1,
dadurch gekennzeichnet,
daß man mit einer wäßrigen Alkalihypochlorit-Lösung umsetzt.

3. Verfahren nach Anspruch 1,
dadurch gekennzeichnet,
daß man als Lösevermittler Acetonitril verwendet.

4. Verfahren nach Anspruch 1,
dadurch gekennzeichnet,
daß man die Reaktion bei pH 11 bis 14 durchführt.

5. Verfahren nach Anspruch 1,
dadurch gekennzeichnet,
daß man die Reaktion bei einer Temperatur von 0 bis 50 °C, vorzugsweise von 2 bis 25 °C ausführt.

6. Verfahren nach Anspruch 1,
dadurch gekennzeichnet,
daß man Arylmalonsäurediester und Hypochlorit im Molverhältnis 1 : 1 bis 1 : 5 umsetzt.

7. Verfahren nach Anspruch 6,
dadurch gekennzeichnet,
daß das Molverhältnis bei 1 : 1 bis 1 : 2 und vorzugsweise bei 1 : 1,05 bis 1 : 1,2 liegt.

## Claims

1. A process for preparing 2-aryl-2-chloromalonic diesters from 2-arylmalonic diesters, characterized in that the 2-arylmalonic diesters are reacted with aqueous hypochlorite in the presence of an inert solubilizer, selected from the group consisting of acetonitrile, propionitrile or butyronitrile, at pH 8 to 14.

2. A process according to claim 1, characterized in that the reaction is carried out using an aqueous alkali metal hypochlorite solution.

3. A process according to claim 1, characterized in that the solubilizer used is acetonitrile.

4. A process according to claim 1, characterized in that the reaction is carried out at pH 11 to 14.

5. A process according to claim 1, characterized in that the reaction is carried out at a temperature of from 0 to 50°C, preferably from 2 to 25°C.

6. A process according to claim 1, characterized in that arylmalonic diester and hypochlorite are reacted in a molar ratio of from 1:1 to 1:5.

7. A process according to claim 6, characterized in that the molar ratio is from 1:1 to 1:2 and preferably from 1:1.05 to 1:1.2.

## Revendications

1. Procédé de fabrication de diesters d'acides 2-aryl-2-chloromaloniques à partir d'esters d'acides 2-aryl-maloniques,
caractérisé en ce qu'
on fait réagir les diesters d'acides 2-aryl-maloniques avec de l'hypochlorite aqueux en présence d'un solvant intermédiaire inerte choisi parmi l'acétonitrile, le propionitrile ou le butyronitrile à pH 8 à 14.

2. Procédé selon la revendication 1,
caractérisé en ce qu'
on procède à la réaction avec une solution aqueuse d'hypochlorite de métal alcalin.

3. Procédé selon la revendication 1,
caractérisé en ce qu'
on utilise comme solvant intermédiaire l'acétonitrile.

4. Procédé selon la revendication 1,
caractérisé en ce qu'
on conduit la réaction à pH 11 à 14.

5. Procédé selon la revendication 1,
caractérisé en ce qu'
on conduit la réaction à une température de 0 à 50°C, de préférence de 2 à 25°C.

6. Procédé selon la revendication 1,
caractérisé en ce qu'
on fait réagir le diester d'acide arylmalonique et l'hypochlorite dans un rapport molaire de 1:1 à 1:5.

7. Procédé selon la revendication 6,
caractérisé en ce que
le rapport molaire se situe à 1:1 à 1:2 et de préférence à 1:0,5 à 1:1,2.
